(19) Europäisches Patentamt
European Patent Office
Office européen des brevets

(11) Publication number: 0 024 779
B1

(12) **EUROPEAN PATENT SPECIFICATION**

(45) Date of publication of patent specification: 17.10.84

(21) Application number: 80300257.5

(22) Date of filing: 29.01.80

(51) Int. Cl.³: A 61 F 5/47, A 61 K 9/02, A 61 K 31/155, C 07 C 123/00, C 07 C 129/00

# (54) Drugs for use with intrauterine devices and intrauterine devices containing said drugs.

(30) Priority: 09.07.79 US 55903

(43) Date of publication of application: 11.03.81 Bulletin 81/10

(45) Publication of the grant of the patent: 17.10.84 Bulletin 84/42

(84) Designated Contracting States: DE FR GB IT NL SE

(56) References cited:
DE-A-2 247 949
FR-A-2 282 262
GB-A- 376 806
GB-A- 516 289
GB-A- 938 042

DERWENT JAPANESE PATENT REPORT, vol. T/11, no. 28, 1972, London GB

UNLISTED DRUGS, vol. 24, april 1972, page 53 Chatham, New Jersey US * Abstract C *

(73) Proprietor: Shaw, Seth Thomas, Jr.
30036 Via Borica Rancho Palos Verdes
California (US)

(72) Inventor: Shaw, Seth Thomas, Jr.
30036 Via Borica Rancho Palos Verdes
California (US)

(74) Representative: Coxon, Philip et al
Eric Potter & Clarkson 14 Oxford Street
Nottingham NG1 5BP (GB)

(56) References cited:
DIE PHARMAZIE, vol. 25, September 1970, Berlin DD F. MARKWARDT et al.: "Hemmung der Trombin-, Plasmin- und Trypsinwirkung durch Alkyl- und Alkoxybenzamidine", pages 551-554

"Burger's Medicinal Chemistry", 4th edn. (1979), part 2, pages 57-9, 448-9, 458-9,464, 467, 556-7

Organisch Chemische Arzneimittel und ihre Synonyma", 5th edn. (1978) Vol. 1, entries 304, 483, 880, 1161, 1340, 2111 and 2670

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 024 779 B1

(56) References cited:

**CHEMICAL ABSTRACTS, vol. 74, no. 15, 12-04-1971, page 306, column 2, abstract 74.827z Columbus, Ohio, US L.J. ZANEVELD: "Synthetic enzyme inhibitors as antifertility agents"**

**UNLISTED DRUGS, vol. 24, April 1972, page 57 Chatham New Jersey US**

**DIE PHARMAZIE, vol. 28, May 1973 Berlin DD E. WAGNER et al.: "Synthese antiproteolytisch wirksamer Ester von Guanidinobenzoesäuren und Guanidinomethylbenzoesäuren", pages 203-296**

**Description**

This invention relates to drugs in conjunction with intrauterine devices and more particularly concerns amidines, in particular aromatic monoamidines, aromatic diamidines and non-aromatic diamidines, and guanidines, in particular aromatic monoguanidines, aromatic diguanidines, non-aromatic monoguanidines, and non-aromatic diguanidines, in conjunction with an intrauterine device. The drug which, when released at a predetermined controlled rate from an intrauterine device, provides an anti-fibrinolytic action, an anti-proteolytic action to reduce and/or eliminate metrorrhagia and menorrhagia as well as enhancing the contraceptive effect of the intrauterine device itself and, it is believed, reducing or eliminating the pain and cramps associated with wearing an intrauterine device and reducing or eliminating undesired expulsion of the intrauterine device. The guanidine may be utilized alone as the drug or mixed with an amidine such as an aromatic monoamidine, aromatic diamidine, or non-aromatic diamidine.

Many forms and configurations of intrauterine devices designed to prevent conception in the female have heretofore been utilized. Such devices have been provided in a variety of shapes, such as the "T" device shown in U.S. Patent 3,533,406, the Loop, such as shown in Patent 3,200,815, a "Y" configuration, generally termed a "Ypsilon" configuration, a ring or modified ring such as the Ota ring, and many modifications thereto, including flat, leaf-like members between various segments of the intrauterine device. Such intrauterine devices which were not provided with any medications associated therewith depended upon their presence in the uterus to prevent conception.

Further, other intrauterine devices (IUDs) have incorporated a controlled release rate medication or drug therein to further aid the anticonceptive action thereof. Such medicated IUDs have generally employed copper or progesterone as the contraceptive or antifertility agent. However, it has been found that copper-releasing intrauterine devices, as well as non-medicated intrauterine devices still resulted in pain and cramping to the wearer, as well as metrorrhagia and menorrhagia. Consequently, the excessive uterine hemorrage, with or without pain, continues to be a leading cause for this type of intrauterine device removal. The progesterone-releasing intrauterine devices are associated with significantly less bleeding than other devices but they appear to be associated with a serious complication apparently produced by the release of progesterone. This complication is ectopic pregnancy.

Nevertheless, the general convenience and safety of intrauterine devices continues to give hope that the IUD may one day provide an ideal method for worldwide population control, since it has been found, statistically, that intrauterine devices can provide effective contraception in a 98—99% range of effectivity, they do not require conscious effort, are less subject to human failings than any other type of contraceptive, their antifertility effect is completely reversible, they have minimal, if any systemic effect, and their effect is confined essentially to the uterus. However, it is belived that even greater antifertility effect can be achieved by utilizing other anticonceptic agents with an IUD, which agents do not have the serious detrimental side effects noted above.

Consequently, there has been a need for improved medicate intrauterine devices providing greater antifertility effect and in which the side effects of pain, metrorrhagia and/or menorrhagia are reduced or eliminated, and which are not associated with other serious side effects such as ectopic pregnancy.

While the inflammatory response of the endometrium to intrauterine devices has heretofore been known, I have discovered that the chronic response of the endometrium to long-term intrauterine device exposure is more a humoral type of reaction (accompanied by increased vascular permeability with edema and interstitial hemorrhage) than the immunologic or cellular type of response (accompanied by infiltration of immune complexes or of leukocytes, such as plasma cells or neutrophils). However, I have found that there are defects in small endometrial vessels which suggest damage caused by mechanical distortion of the uterine tissues. The defects generally lack hemostatic plugs of platelets and/or fibrin. Further, there is evidence that fibrinolysis is activated in the uterus in response to the presence of an intrauterine device. This activation could result in blockage of normal hemostatic reaction at several levels in the coagulation system. Further, it may initiate, aid or aggravate humoral inflammation by any one or all of the following mechanisms:

1. Activation of the complement system and histamine release;
2. Activation of prekallikrein; and
3. Release of fibrin degradation fragments.

Histamine can cause vascular dilation and increase vascular permeability. Kallikrein (activated prekallikrein) releases bradykinin which can have an effect similar to histamine and may also cause cramping and pain. Fibrin degradation fragments may enhance the vascular effects of histamine and bradykinin. Combined with distortion of the endometrium caused by myometrial contractility around the relatively inelastic or unyielding IUD, which may also be associated with increased prostaglandin synthesis and release, it may be predicted that excessive bleeding from leaky or broken vessels will occur. For these reasons, incorporation into medicated IUD devices of potent inhibitors of plasminogen activation and plasmin activity (fibrinolytic activity) for the purposes of intrauterine release over an extended time period can provide an alleviation of the aforesaid undesired effects.

It has also heretofore been found that IUD associated uterine hemorrhage can be alleviated by the systemic (oral) intake of the fibrinolytic inhibitors epsilon aminocaproic acid (EACA) and tranexamic acid. I have also demonstrated that and EACA loaded IUD inserted into the uterus of rhesus monkeys provides an ameliorative effect on menstral blood loss, and there was no apparent systemic effect by such medicated devices on fibrinolytic activity in these animals. However, neither EACA nor tranexamic acid would appear to be satisfactory agents for long-time intrauterine medication. First, they are not highly potent anti-fibrinolytic agents and would have to be delivered at a rather high rate into the uterine cavity. Thus, a drug loaded IUD would become exhausted of its medication in a short period of time, or would require an unacceptably large size of device. In addition, EACA and tranexamic acid are small molecules which are highly diffusible and water soluble. Therefore, intrauterine release thereof from a medicated intrauterine device at a steady, constant rate is difficult to control and effective concentration inside the uterus difficult to maintain. Consequently, inhibitor concentrations of either EACA and tranexamic acid of between $1 \times 10^{-3}$ and $1 \times 10^{-4}$ Mol/liter, which is the concentration of these drugs required to be effective, respectively, over a prolonged period of time is generally not achievable considering the amount of medication which is feasible to load into an IUD and considering the diffusion and solubility properties of these compounds and the rate of water turnover inside the uterus.

While there heretofore has been some indication that certain compounds used for treatment of protozoal, bacterial and fungal infections may have anti-fibrinolytic properties, there has not heretofore been any indication of anti-fertility action of these compounds added to an intrauterine device. These compounds may be generally defined as the aromatic amidines, and in particular, the aromatic diamidines. However, heretofore, it has not been specifically recognized that their anti-fibrinolytic action inside the uterus can alleviate the metrorrhagia and menorrhagia. Further, even though such metrorrhagia and menorrhagia may be alleviated, the pain and cramps associated with intrauterine devices could still remain a major drawback to effective extensive use of medicated intrauterine devices as a population control technique.

Additionally, in many prior art IUDs, expulsion thereof is a somewhat frequent occurrence. Such undesired expulsion is another drawback of prior art IUDs.

In addition to the above-mentioned types of intrauterine devices, there have also heretofore been provided intrauterine devices in which all or a part of the device is hollow and thus the device has walls defining the cavity. Such a device is shown, for example, in Patent 3,896,819 and other types of such devices are shown, for example, in Patents 3,710,795 in which the cavity is filled with a solid matrix having a drug therein, and other prior art patents. Similarly, there have heretofore been proposed inflatable intrauterine devices in which the walls of the intrauterine device are flexible and it is inserted into the uterus in uninflated condition and subsequently expanded.

However, in none of the prior art devices has there heretofore been provided a drug releasable at a controlled rate over an extended period of time which drug provides not only an antiproteolytic action but an enhanced contraceptive action. Accordingly, there has long been a need for an intrauterine device which can provide the above desiderata.

Additionally, in many prior art IUDs, expulsion thereof is a somewhat frequent occurrence. Such undesired expulsion is another drawback of prior art IUDs.

Consequently, there has long been a need for a medicated intrauterine device which not only enhances the anti-fertility action of the IUD but also provides reduction or elimination of metrorrhagia or menorrhagia for an extended period of time, as well as decreasing the pain and cramps associated with wearing an intrauterine device, as well as decreasing the tendency of expulsion thereof.

The contraceptive effects of certain guanidine and amidine compounds and groups of compounds are disclosed in:

"CHEMICAL ABSTRACTS", Vol. 74, abstract No. 74 827 z
"UNLISTED DRUGS", Vol. 24, page 57, abstract a and
"UNLISTED DRUGS", Vol. 24, page 53, abstract c.
"DIE PHARMAZIE", Vol. 25, pages 551—554 and Vol. 28, pages 293—6 disclose anti proteolytic properties of guanidine and amidine derivatives.

It is an object of the present invention to provide an improved intrauterine device.

According to the present invention I provide a medicated intrauterine device characterized in that the medicament is a drug comprising one or more of an aromatic monoamidine, an aromatic diamidine, a nonaromatic diamidine, a guanidine or an ester or salt thereof, said drug being releasable at a rate of 50 to 200 $\mu$g per day and said released amount being such as to produce an anti-proteolytic, an anti-fibrinolytic and anti-conceptive effect.

Preferably the drug comprises:

(a) a mixture of an amidine and a guanidine;
(b) a mixture of more than one amidine and a guanidine;
(c) a mixture of an amidine and more than one guanidine;
(d) a mixture of more than one amidine and more than one guanidine;

(e) a guanidine; and
(f) a mixture of more than one guanidine.

The antiproteolytic action and, in particular, the anti-fibrinolytic action of the aromatic monoamidines, aromatic diamidines and non-aromatic diamidines can provide a reduction in metrorrhagia and menorrhagia because of the particular characteristics associated with the reaction of the endometrium and/or the fluid of the uterus to the presence of an intrauterine device. Further, it is believed that inhibition of other proteolytic systems in the endometram and/or muscle wall of the uterus can reduce and/or eliminate the pain and cramps associated with wearing an intrauterine device, as well as minimizing the risk of expulsion thereof. The aromatic amidines and, in particular, the aromatic monoamidines, aromatic diamidines, and non-aromatic diamidines, have been found to possess the desired properties, due to the anti-fibrinolytic and other antiproteolytic effect thereof, to reduce or eliminate metrorrhagia and/or menorrhagia.

Additionally, I have discovered that there is a surprising and unexpected result in utilization of aromatic diamidines with intrauterine devices in that they enhance the anti-fertility effect of the IUD. That is, they may cause a greater contraceptive effect than has heretofore been obtainable with prior art IUDs of either the plain or medicated type. There has not, heretofore, been any recognition that the guanidines would also have an antiproteolytic action, an anti-fibrinolytic, an anti-bleeding action, or an anti-fertility action when controllably released from an IUD. I have discovered, however, that the guanidines, in addition to the amidines, do have such properties and, it is believed, may have even more potent effects.

Thus, I have also discovered that there is a surprising and unexpected result in utilization of guanidines with intrauterine devices in that they may decrease IUD induced uterine bleeding and enhance the anti-fertility effect of the IUD by providing an anti-proteolytic and, particularly, an anti-fibrinolytic action in the uterus. Each treated IUD, therefore, may, additionally, cause a greater contraceptive effect than has heretofore been obtainable with the above-mentioned prior art IUDs of either the plain or medicated type. This unexpected result, it is believed, is achieved by the mechanism of the aromatic diamidine or the guanidine acting upon the fertilised egg or the blastocyst (preimplantation embryo) to cause it to degenerate. The aromatic diamidine could, in addition, act on the sperm to either kill or render them ineffective in fertilization.

Further, it is believed, that certain anti-proteolytic action of the aromatic diamidines and/or the guanidines could reduce or eliminate the pain and cramps often associated with wearing an IUD.

The drug may be incorporated in a uterus insertable IUD body member. The body member may be of any desired shape or configuration of an intrauterine device, such as those heretofore utilized, or any other suitable configuration. The drug may be one or more drugs selected from the class consisting of aromatic monoamidines, aromatic diamidines, non-aromatic diamidines, aromatic monguanidine, aromatic diguanidine, non-aromatic monoguanidine, and non-aromatic diguanidine, or a mixture of one or more guanidines with one or more amidines and is provided with the body member in such a fashion that its release rate over an extended period of time is controlled within predetermined limits. As utilized herein, the term "drug" refers to either a single one of the above-mentioned amidines, guanidines or a mixture of more than one of the amidines and/or guanidines.

The body member of the intrauterine device is usually a polymer matrix fabricated in any of the above-mentioned shapes or configurations from, for example, polyethylene, and the drug may be supplied as a simple mixture with the polymer matrix. The shape, charge, and other characteristics such as hydrophobicity of the molecule of the drug, as well as the characteristics of the polymer matrix of the body member can be adjusted to determine the rate of drug release from the device when placed within the uterus to achieve a desired rate of emission of the drug over a predetermined time period by known techniques.

The drug may be provided with a biodegradable polymer or copolymer with, for example, another amidine guanidine or epsilon aminocaproic acid, and mixed with the supporting polymer matrix of the body member.

The drug may be provided in a biodegradable polymer or copolymer, as shown, and bonded covalently or non-covalently to the polymer matrix of the device either within or on the surface of the polymer matrix of the body member.

Additionally, the above-mentioned possibilities may be combined with a surface coating on the body member wherein the surface coating comprises a biodegradable cross-linked polymer or copolymer of the drug bonded covalently to the surface. Such embodiments may provide a soft hydrogel coating which enhances the tolerance of the walls of the uterus to enhance the retention of the medicated intrauterine device in the uterus during the time period soon after insertion. It has been found that the undesired expulsion often occurs during this time period.

Additionally, and optionally in combination with any of the above possibilities, a coating is provided on some or all of the surfaces of the body member. The coating may be covalently bonded to the surface of the body member and consists of a non-biodegradable monomer, dimer, oligomer, or cross-linked polymer of the drug. This provides a prolonged surface effect for reducing deleterious effects on the uterine wall, as well as providing the desired prolonged release of the drug from the body member.

The bleeding of the endometrium in contact with the intrauterine device is at the surface of the endometrium. The inhibition of plasminogen activator and plasmin by solid-phase enzyme inhibitors such as the surface linked drugs described herein constantly during the duration of wearing of the intrauterine device could lead to a lessening of the bleeding at the interface between the endometrium and the intrauterine device.

The surface of the body member of any one of the above-defined possibilities may be partially covered by copper such as wire, plating or the like to provide additional anti-conceptive action for the device.

The drug may be utilized either in its base form, or as certain esters such as isethionate, or as certain salts, such as hydrochloride or phosphate, depending upon the degree of solubility desired in the uterine fluid for control of release rate and tissue uptake of the drug, as well as enhancing the effectiveness of the particular compound employed.

The drug may be provided in a concentrated form in a cavity of the IUD which is permeable to the drug. The cavity may be in a semi-rigid form or flexible walled form.

As noted above, the present invention relates to medicated intrauterine devices wherein the preselected drug is utilized in conjunction with an intrauterine device. As utilized herein and in the appended claims, the term "drug" refers to one or a mixture of more than one of preselected compounds which, in the present invention are the amidines and in particular the aromatic monoamidines aromatic diamidines and non-aromatic diamidines, and the guanidines and in particular the aromatic monoguanidines, the aromatic diguanidines and non-aromatic monoguanidines, and the non-aromatic diguanidines.

The aromatic amidines may be an aromatic monoamidine of the general formula:

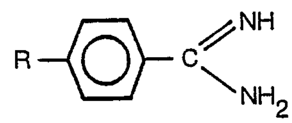

wherein: R is a carbon chain or an aromatic group or an aromatic group with or without other elements, or, as preferred for utilization in the invention herein, an aromatic diamidine of the general formula:

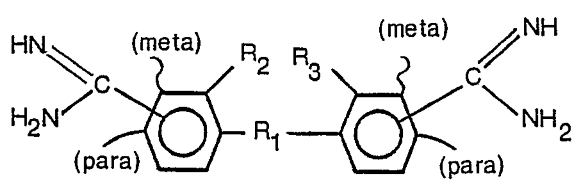

in which each amidine group

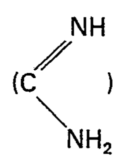

may be substituted in either a meta or para position with respect to $R_1$.

wherein: $R_1$ is generally a carbon chain with or without ether bonds to the benzene rings;
$R_2$ and $R_3$ can be hydrogen, chlorine, bromine, iodine, hydroxyl group, alkyl, or other group; and

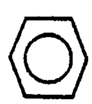

represents the benzene ring.

The aromatic guanidines may be an aromatic monoguanidine of the general formula:

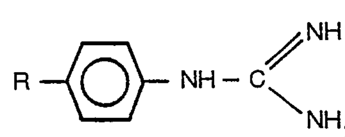

wherein: R is a carbon chain with or without other elements (such as hydrogen, nitrogen, oxygen, sulfer, etc.); an aromatic group (such as benzene) with or without additional carbons, carbon chains, and other elements; a cyclic non-aromatic group (such as cyclohexane) with or without additional carbons, carbon chains, and other elements; or any of the above in combination; and

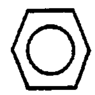

represents the benzene ring.

As preferred for utilization in the invention herein, there may be utilized an aromatic diguanidine of the general formula:

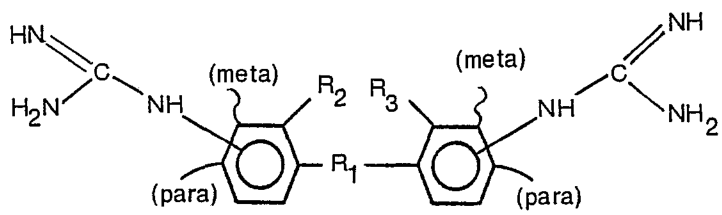

in which each guanidine group:

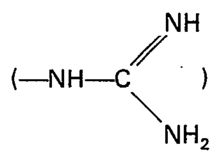

may be substituted in either a meta or para position with respect to $R_1$, and wherein:

$R_1$ is generally a hydrocarbon chain with or without ether or ester bonds to the benzene rings; $R_2$ and $R_3$ can be hydrogen, chlorine, bromine, iodine, hydroxyl group, alkyl, or other group; and

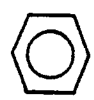

represents the benzene ring.

Table I below lists particular aromatic diamidines useful in the practice of the present invention.

It is understood that the series of examples of aromatic diamidines in Table I, below, will also exemplify the aromatic diguanidines in every respect except that for the latter class of compounds guanidino groups:

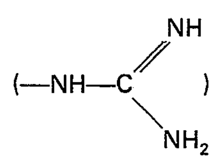

are substituted for amidino groups:

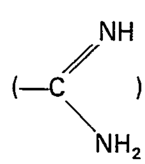

Two examples of aromatic monoguanidines are the following:

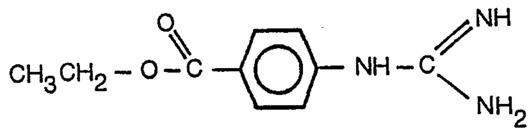

ethyl-p-guanidinobenzoate

and

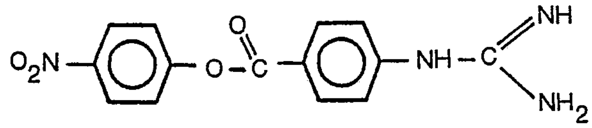

p-nitrophenyl-p'-guanidinobenzoate (commonly named NPGB).

Two examples of aromatic diguanidines are the following:

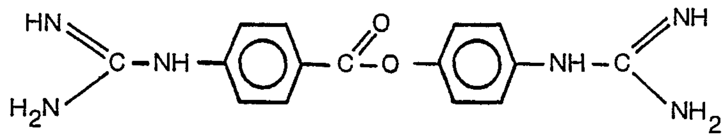

p-guanidinophenyl-p' guanidinobenzoate

and

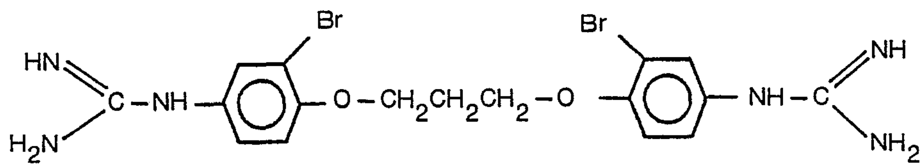

1,3 bis (2-bromo-4-guanidinophenoxy) propane.

{This last compound is analogous to dibromopropamidine (Table I) except that it is a diguanidine rather than a diamidine by virtue of the two guanidino groups:

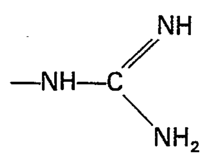

at both extremities of the molecule in place of the two amidino groups:

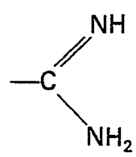

This diguanidine is assigned a chemical name in this application rather than a common or trivial name (such as "dibromopropaguanidine") because the compound and its analogs are not in common use and have not been previously given common names in the scientific literature.}.

The non-aromatic guanidines may be a non-aromatic monoguanidine of the general formula:

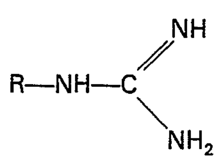

wherein R may represent a carbon chain with or without other elements (such as hydrogen, nitrogen, oxygen, sulfer, etc.); a cyclic non-aromatic group (such as cyclohexane) with or without additional carbons, carbon chains, and other elements; or any of the above in combination.

As preferred for utilization in the invention herein, there may be utilized a non-aromatic diguanidine of the general formula:

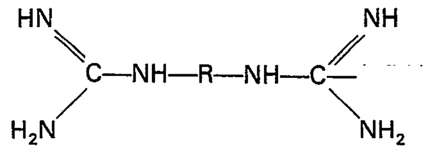

R may represent a carbon chain with or without other elements (such as hydrogen, nitrogen, oxygen, sulfur, etc.); a cyclic aromatic group (such as cyclohexane) with or without additional carbons, carbon chains, and other elements; or any of the above in combination.

An example of a non-aromatic monoguanidine is the following:

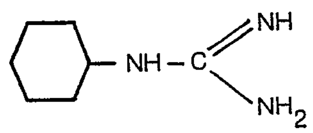

guanidinocyclohexane.

An example of a non-aromatic diguanidine is the following:

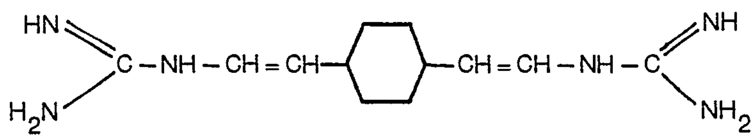

1,4-di(2-guanidinovinyl)cyclohexane.

In the above,

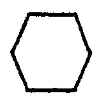

represents cyclohexane.

TABLE 1

AROMATIC DIAMIDINES

| Drug Name | $R_1$ Carbon Chain | $R_2$ | $R_3$ | Relative Potency |
|---|---|---|---|---|
| Dibromopropamidine | $C_3H_6$ | Br | Br | 1.0 |
| Phenamidine | — | H | H | 0.2 |
| Octamidine | $C_8H_{16}$ | H | H | 2.6 |
| m-Pentamidine | $C_5H_{10}$ | H | H | 0.6 |
| Hexamidine | $C_6H_{12}$ | H | H | 1.6 |
| Dichlorohexamidine | $C_6H_{12}$ | Cl | Cl | 1.9 |
| Pentamidine | $C_5H_{10}$ | H | H | 2.4 |
| Monoiodohexamidine | $C_6H_{12}$ | I | H | 4.4 |
| Dibromopentamidine | $C_5H_{10}$ | Br | Br | 3.6 |
| Propamidine | $C_3H_6$ | H | H | 1.2 |

TABLE 1 (Cont'd).

AROMATIC DIAMIDINES

| Drug Name | $R_1$ Carbon Chain | $R_2$ | $R_3$ | Relative Potency |
|---|---|---|---|---|
| Heptamidine | $C_7H_{14}$ | H | H | 1.9 |
| Diiodopentamidine | $C_5H_{10}$ | I | I | 6.8 |
| Diiodohexamidine | $C_6H_{12}$ | I | I | 7.5 |
| Butamidine | $C_4H_8$ | H | H | |
| Monochloropropamidine | $C_3H_6$ | Cl | H | |
| Monochlorobutamidine | $C_4H_8$ | Cl | H | |
| Monochloropentamidine | $C_5H_{10}$ | Cl | H | |
| Monochlorohexamidine | $C_6H_{12}$ | Cl | H | |
| Monochloroheptamidine | $C_7H_{14}$ | Cl | H | |
| Monochloroctamidine | $C_8H_{16}$ | Cl | H | |
| Monochlorononamidine | $C_9H_{18}$ | Cl | H | |
| Monobromopropamidine | $C_3H_6$ | Br | H | |
| Monobromofutamidine | $C_4H_8$ | Br | H | |
| Monobromopentamidine | $C_5H_{10}$ | Br | H | |
| Monobromohexamidine | $C_6H_{12}$ | Br | H | |
| Monobromoheptamidine | $C_7H_{14}$ | Br | H | |
| Monobromoctamidine | $C_8H_{16}$ | Br | H | |
| Monobromononamidine | $C_9H_{18}$ | Br | H | |
| Monoiodopropamidine | $C_3H_6$ | I | H | |
| Monoidobutamidine | $C_4H_6$ | I | H | |
| Monoiodopentamidine | $C_5H_{10}$ | I | H | |
| Monoiodohexamidine | $C_6H_{12}$ | I | H | |
| Monoiodoheptamidine | $C_7H_{14}$ | I | H | |
| Monoiodoctamidine | $C_8H_{16}$ | I | H | |
| Monoiodononamidine | $C_9H_{18}$ | I | H | |
| Dichloropropamidine | $C_3H_6$ | Cl | Cl | |
| Dichlorobutamidine | $C_4H_8$ | Cl | Cl | |
| Dichloropentamidine | $C_5H_{10}$ | Cl | Cl | |
| Dichlorohexamidine | $C_6H_{12}$ | Cl | Cl | |

TABLE 1 (Cont'd).

AROMATIC DIAMIDINES

| Drug Name | $R_1$ Carbon Chain | $R_2$ | $R_3$ | Relative Potency |
|---|---|---|---|---|
| Dichloroheptamidine | $C_7H_{14}$ | Cl | Cl | |
| Dichloroctamidine | $C_5H_{16}$ | Cl | Cl | |
| Dichlorononamidine | $C_9H_{19}$ | Cl | Cl | |
| Dibromopropamidine (already listed) | $C_3H_6$ | Br | Br | |
| Dibromobutamidine | $C_4H_8$ | Br | Br | |
| Dibromopentamidine | $C_5H_{10}$ | Br | Br | |
| Dibromohexamidine | $C_6H_{12}$ | Br | Br | |
| Dibromoheptamidine | $C_7H_{14}$ | Br | Br | |
| Dibromoctamidine | $C_8H_{16}$ | Br | Br | |
| Dibromononamidine | $C_9H_{18}$ | Br | Br | |
| Diiodopropamidine | $C_3H_6$ | I | I | |
| Diiodobutamidine | $C_4H_8$ | I | I | |
| Diiodopentamidine | $C_5H_{10}$ | I | I | |
| Dioodohexamidine | $C_6H_{12}$ | I | I | |
| Diiodoheptamidine | $C_7H_{14}$ | I | I | |
| Diiodooctamidine | $C_8H_{16}$ | I | I | |
| Diiodononamidine | $C_9H_{18}$ | I | I | |
| Monochloromonobromo-propamidine | $C_3H_6$ | Cl | Br | |
| Monochloromonobromo-butamidine | $C_4H_8$ | Cl | Br | |
| Monochloromonobromo-pentamidine | $C_5H_{10}$ | Cl | Br | |
| Monochloromonobromo-hexamidine | $C_6H_{12}$ | Cl | Br | |
| Monochloromonobromo-heptamidine | $C_7H_{14}$ | Cl | Br | |
| Monochloromonobromo-octamidine | $C_8H_{16}$ | Cl | Br | |
| Monochloromonobromo-nonamidine | $C_9H_{18}$ | Cl | Br | |

TABLE 1 (Cont'd).

AROMATIC DIAMIDINES

| Drug Name | $R_1$ Carbon Chain | $R_2$ | $R_3$ | Relative Potency |
|---|---|---|---|---|
| Monochloromonoiodo-propamidine | $C_3H_6$ | Cl | I | |
| Monochloromonoiodo-butamidine | $C_4H_8$ | Cl | I | |
| Monochloromonoiodo-pentamidine | $C_5H_{10}$ | Cl | I | |
| Monochloromonoiodo-hexamidine | $C_6H_{12}$ | Cl | I | |
| Monochloromonoiido-heptamidine | $C_7H_{14}$ | Cl | I | |
| Monochloromonoiodo-octamidine | $C_8H_{16}$ | Cl | I | |
| Monochloromonoiodo-nonamidine | $C_9H_{18}$ | Cl | I | |
| Monobromomonoiodo-propamidine | $C_3H_6$ | Br | I | |
| Monobromomonoiodo-butamidine | $C_4H_8$ | Br | I | |
| Monobromomonoiodo-pentamidine | $C_5H_{10}$ | Br | I | |
| Monobromomonoiodo-hexamidine | $C_6H_{12}$ | Br | I | |
| Monobromomonoiodo-heptamidine | $C_7H_{14}$ | Br | I | |
| Monobromomonoiodo-octamidine | $C_8H_{16}$ | Br | I | |
| Monobromomonoiodo-nonamidine | $C_9H_{18}$ | Br | I | |

In addition to the specified aromatic diamidines listed in Table I, other aromatic diamidines, aromatic monomidines and non-aromatic diamidines may also be utilized in accordance with the present invention.

Further, in addition to the aromatic diguanidines, which, as noted above, are similar to the aromatic diamidines listed in Table I except for the substitution of the guanidine group for the amidine group in the drug and which, when trivial names have been assigned thereto will have trivial names similar to those shown in Table I other aromatic diguanidines, aromatic monoguanidines, non-aromatic monoguanidines and non-aromatic diguanidines may also be utilized in accordance with the present invention.

Further, it has been found that the following compounds are also useful in the practice of the present invention:

| DRUG | SPECIFIC FORMULA |
|---|---|
| 3,8-Di(*m*-amidinophenyldiazoamino)-5-ethyl-6-phenylphenanthridinium chloride dihydrochloride hydrate (aromatic diamidine) | 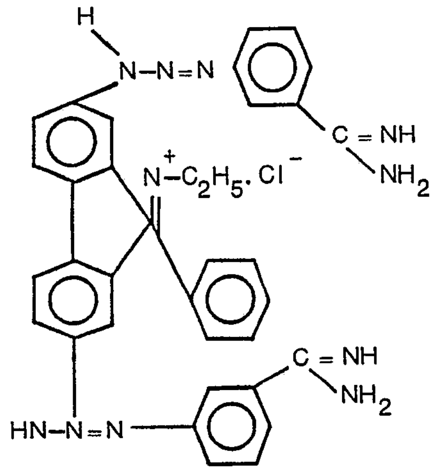 |
| 8-(m-amidophenyldiazoamino)-3-amino-5-ethyl-6-phenylphenanthridinium chloride (aromatic monoamidine) | 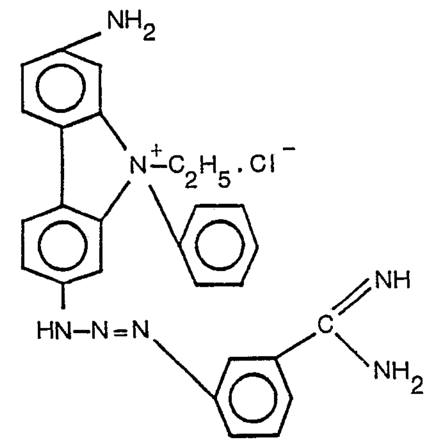 |
| 1,4-di (p-amidinophenoxy) cyclohexane (aromatic diamidine) | 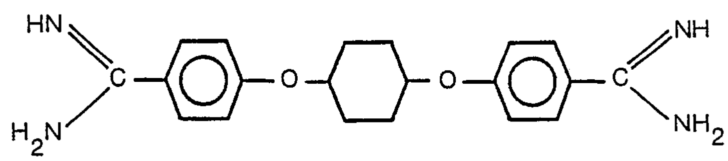 |
| 1,4-di (2-amidinovinyl) cyclohexane (nonaromatic diamidine) | 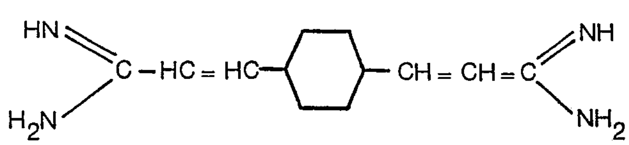 |

The relative potency shown in Table I is expressed in relationship to dibromopropamidine, which has been discovered to be a highly potent fibrinolytic inhibitor. The numerical values are expressed as a reciprocal of the concentration of the drug producing the equivalent inhibition to the dibromopropamidine. Where no values for relative potency are listed such values have not been specifically determined.

The exact relative potency for the guanidines has not yet been completely determined. However, those skilled in the art may rapidly determine the relative potency for any particular guanidine selected.

When utilized as a mixture with the polymer matrix of a solid IUD (that is, one without a cavity) the ratio of the drug may be, for example, on the order of 10% to 50% drug by weight of the IUD, depending upon the potency of the drug and the particular polymer matrix of the IUD. Additionally, the

shape, charge, and other characteristics of the drug molecule, such as its hydrophobicity, as well as similar characteristics of the polymer matrix, may be varied as desired to select the particular release rate of the drug from IUD when placed within the uterus.

The drug may be provided as a biodegradable polymer or copolymer and mixed into the supporting polymer matrix of the IUD with selections of characteristics as defined above.

The drug is provided in a biodegradable polymer or copolymer form and it is covalently bonded to the polymer matrix of the IUD either or both within the IUD or on the surface thereof.

In any of the possibilities described above, there may also be provided a biodegradable cross-linked polymer or copolymer coating of the drug bonded covalently to the surface of the polymer matrix of the IUD in order to provide a soft hydrogel coating thereover. Such a coating is likely to be particularly effective in aiding retention of the intrauterine device in the uterus during the time period soon after insertion thereof. The coating may be provided for some or all of the surface.

The drug may be provided in a non-biodegradable monomer, dimer, or oligomer or a cross-linked polymer on the outer surface of the polymer matrix of the IUD in order to maintain a prolonged hydrogel effect at the interface between the device and the walls of the uterus, in addition to the predetermined release rate of the drug from the IUD. Since the bleeding of the endometrium is at the interface between the endometrium and the intrauterine device, the solid phase enzyme inhibition provided by the drug at the point of contact between the endometrium and the intrauterine device can reduce the bleeding associated with utilizing an intrauterine device.

Further, since copper release has also been proven anticonceptive in IUDs, some of the surface of the IUD may be provided with a coating of copper such as wire plating or the like. However, of course, such coating should not completely cover the IUD since that would prevent drug release or exposure to the uterine wall.

It has been found that the drugs provide an anti-conceptive effect. It is believed that this effect, which should enhance the anti-conceptive effect of the intrauterine device itself, is due to the activity of the drug and its action on the very early embryo and possibly on the sperm.

Further, it is believed yet an additional unexpected and surprising result may be obtained due to the anti-proteolytic action of the drug. This effect is a reduction in the pain and/or cramps and expulsion heretofore associated with utilization of intrauterine devices including medicated IUDs.

The range of concentrations necessary to provide the desired effects mentioned above depend, of course, upon the particular drug or combinations selected. For example, for dibromopropamidine introduced into the uterine cavity and endometrial tissue water, and with an endometrial water turnover rate of 200 milliliters per day and with complete distribution of the drug in the endometrial water turned over, an intrauterine release rate of 50 to 200 $\mu$g per day would be expected to produce a concentration of dibromopropamidine in the range of 0.5 to $2.0 \times 10^{-6}$ moles per litre in endometrial water. Since, in general, there will be less than complete distribution of the drug into the endometrial water turned over each day, the concentration of the drug in the uterine cavity could reach much higher levels; for example, on the order of $10^{-6}$ to $10^{-4}$ moles per litre. This concentration range is sufficient to provide both the anti-fibrinolytic effects, as well as the anti-conceptive or anti-fertility effects desired, and also, it is believed the reduction in pain; cramps and expulsion. With the above release rate (50—200 $\mu$g per day) and the known sizes of intrauterine devices currently available, and the amount of drug which can be incorporated into such devices, an effective life span of, for example, at least one to three years can be provided for such medicated devices.

The drug may be in a concentrated aqueous or organic or non-organic hydrophobic solution within the cavity of an IUD. The cavity may be in a semi-rigid or flexible walled portion thereof. The walls of the IUD defining the cavity are permeable to the drug to allow its entrance into the uterus. The exterior surface of the walls of the IUD may be coated with any of the coatings above mentioned.

The concentration of the drug in the solution required in an inflatable device would range from 50 to 200 mg per ml (5 to 20% by weight). This may be a solution or suspension. The concentration required in a hollow core device would range from 10 to 50% by weight or consist of crystalline drug packed into the hollow core without solvent, or consist of a highly concentrated paste with minimal solvent.

At least one aromatic guanidine, NPGB as identified above, has an anti-fibrinolytic effect on the order of 100 times greater than that of dibromopropamidine (on a molar concentration basis). As little as 0.5 to 2.0 $\mu$g per day release of NPGB from a medicated IUD according to the present invention may be satisfactorily effective. Thus, the estimated range of daily release of the drug according to the present invention from a medicated IUD may be as low as, for example, 0.5 $\mu$g to as high as 200 $\mu$g, depending upon the particular constituents selected for inclusion in the drug. The useful life span of a device releasing, for example, 0.5 $\mu$g per day may greatly exceed three years.

Those skilled in the art, of course, can readily determine the appropriate release rate desired for any drug or combination thereof which may be utilized according to the present invention and, in accordance with known principles, establish the desired release rate thereof to achieve effectiveness.

Medicated intra-uterine devices of particular types as described herein are described in more detail and claimed in my copending European Patent Application Nos: 80300258 and 80300259 (EP—A—24780 and EP—A—24781).

**Claims**

1. A medicated intrauterine device characterised in that the medicament is a drug comprising one or more of an aromatic monoamidine, an aromatic diamidine, a non-aromatic diamidine, a guanidine or an ester or salt thereof, said drug being releasable at a rate of 50 to 200 μg per day and said released amount being such as to produce an anti-proteolytic, an anti-fibrinolytic and anti-conceptive effect.

2. A medicated intrauterine device as defined in Claim 1 wherein said drug further comprises a drug selected from the class of:

(a) a mixture of an amidine and a guanidine;
(b) a mixture of more than one amidine and a guanidine;
(c) a mixture of an amidine and more than one guanidine;
(d) a mixture of more than one amidine and more than one guanidine;
(e) a guanidine; and
(f) a mixture of more than one guanidine.

3. A medicated intrauterine device as defined in Claims 1 or 2 wherein said guanidine is selected from the class consisting of:

(a) aromatic monoguanidines;
(b) aromatic diguanidines;
(c) non-aromatic monoguanidines; and
(d) non-aromatic diguanidines.

4. A medicated intrauterine device as defined in any one of the preceding claims wherein said guanidine is selected from the class consisting of a monoguanidine of the group:

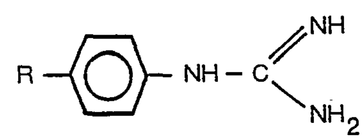

wherein R is selected from the class consisting of:

(a) a carbon chain free of other elements;
(b) a carbon chain with at least one other element;
(c) an aromatic group free of additional carbon atoms, carbon chains and other elements;
(d) an aromatic group with at least one addition selected from the class consisting of carbon atoms, carbon chains and other elements;
(e) a cyclic non-aromatic group free of additional carbon atoms, carbon chains and other elements;
(f) a cyclic non-aromatic group with at least one addition selected from the class consisting of carbon atoms, carbon chains, and other elements; and
(g) a combination of at least two of (a), (b), (c), (d), (e) and (f); and

wherein:

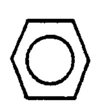

represents the benzene ring;

and/or
said guanidine is selected from the class consisting of aromatic diguanidines of the group:

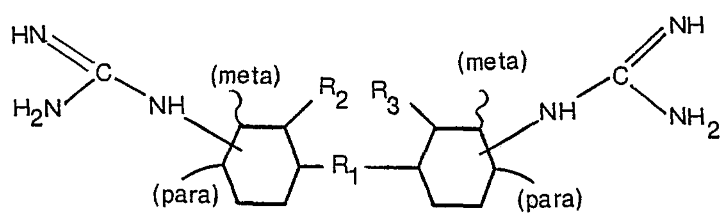

and wherein each guanidine group

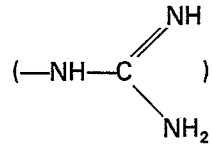

is in one of a meta or para position with respect to $R_1$, and in which:

$R_1$ is selected from the class consisting of:

(a) a hydrocarbon chain free of ether and ester bonds to the benzene ring; and

(b) a hydrocarbon chain having at least one bond selected from the class of ether bonds and ester bonds to the benzene ring;

$R_2$ and $R_3$ are selected from the class consisting of:

hydrogen, chlorine, bromine, iodine, hydroxyl group and alkyl group; and

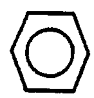

represents benzene ring; and/or said guanidine is a non-aromatic monoguanidine of the group:

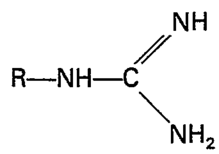

wherein R is selected from the class consisting of:

(a) a carbon chain free of other elements;

(b) a carbon chain with at least one other element;

(c) a cyclic non-aromatic group free of additional carbon atoms, carbon chains and other elements;

(d) a cyclic non-aromatic group with at least one addition selected from the class consisting of carbon atoms, carbon chains and other elements;

(e) a combination of at least two of (a), (b), (c), and (d); and/or

said guanidine is a non-aromatic diguanidine of the group:

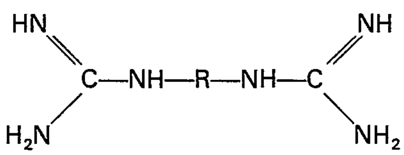

wherein R is selected from the class consisting of:

(a) a carbon chain free of other elements;

(b) a carbon chain with at least one other element;

(c) a cyclic non-aromatic group free of additional carbon atoms, carbon chains and other elements;

(d) a cyclic non-aromatic group with at least one addition selected from the class consisting of carbon atoms, carbon chains and other elements;

(e) a combination of at least two of (a), (b), (c), and (d); and/or

said drug is selected from the class consisting of:

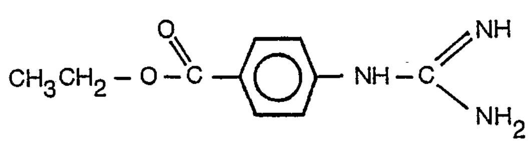

ethyl-p-guanidinobenzoate,

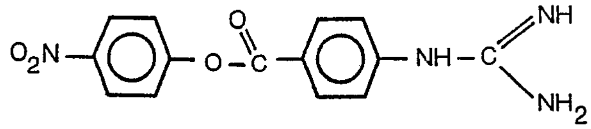

p-nitrophenyl-p′-guanidinobenzoate (commonly named NPGB),

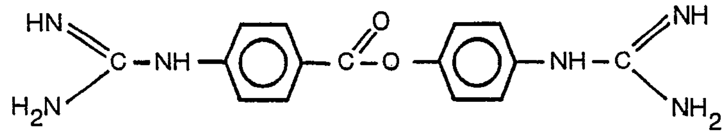

p-guanidinophenyl-p′-guanidinobenzoate,

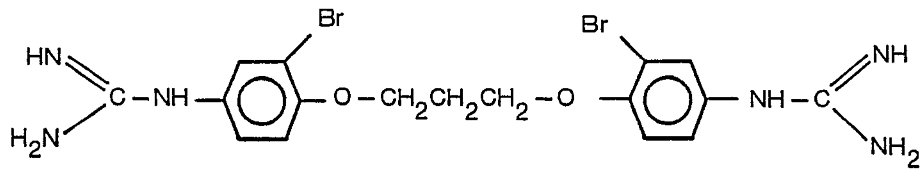

1,3 bis (2-bromo-4-guanidinophenoxy) propane,

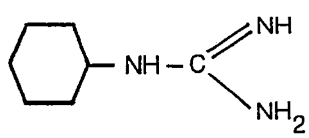

guanidinocyclohexane, and

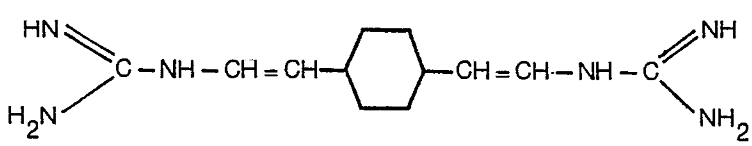

1,4-di(2-guanidinovinyl) cyclohexane.

5. A medicated intrauterine device as defined in any of Claims 1 to 4, the drug being selected from the class consisting of aromatic diamidines of the group

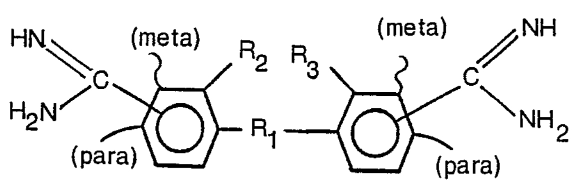

wherein:

$R_1$ is selected from the group consisting of $C_xH_y$; and

$R_2$ and $R_3$ are selected from the group consisting of hydrogen, chlorine, bromine, iodine, hydroxyl group and alkyl groups; and

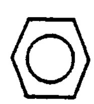

represents the benzene ring.

6. A medicated intrauterine device as defined in any of Claims 1 to 4, the drug being selected from the class consisting of:

3,8-Di(*m*-amidinophenyldiazoamino)-5-ethyl-6-phenylphenanthridinium chloride dihydrochloride hydrate

8-m(m-Aminophenyldiazoamino-3-amino-5-ethyl-6-phenylphenanthridinium chloride, 1,4-di (p-amidinophenoxy) cyclohexane, and 1,4-di (2 amidinovinyl) cyclohexane.

7. A medicated intrauterine device as defined in Claim 6 wherein said amidine is selected from a class consisting of aromatic monoamidines, aromatic diamidines, and non-aromatic diamidines.

**Patentansprüche**

1. Eine ein Medikament aufweisende intra-uterine Vorrichtung, dadurch gekennzeichnet, daß das Medikament ein Arzneimittel ist, das ein oder mehrere aromatische Monoamidine, aromatische Diamidine, nichtaromatische Diamidine, ein Guanidin oder einem Ester oder ein Salz desselben umfaßt, wobei das Arzneimittel mit einer Geschwindigkeit von 50 bis 200 $\mu$g pro Tag freigesetzt wird und die freigesetzte Menge derart ist, daß eine anti-proteolytische, eine anti-fibrinolytische sowie antikonzeptive Wirkung hervorgerufen wird.

2. Eine ein Medikament aufweisende intra-uterine Vorrichtung nach Anspruch 1, bei der das Arzneimittel weiterhin ein Arzneimittel umfaßt, das aus folgender Klasse ausgewählt wird:

(a) ein Gemisch aus einem Amidin und einem Guanidin;
(b) ein Gemisch aus mehr als einem Amidin und einem Guanidin;
(c) ein Gemisch aus einem Amidin und mehr als einem Guanidin;
(d) ein Gemisch aus mehr als einem Amidin und mehr als einem Guanidin;
(e) ein Guanidin und;
(f) ein Gemisch aus mehr als einem Guanidin.

3. Eine ein Medikament aufweisende intra-uterine Vorrichtung nach Anspruch 1 oder 2, bei der das Guanidin ausgewählt wird aus einer Klasse, die besteht aus:

(a) aromatischen Monoguanidinen;
(b) aromatischen Diguanidinen;
(c) nichtaromatischen Monoguanidinen; und
(d) nichtaromatischen Diguanidinen.

4. Eine ein Medikament aufweisende intra-uterine Vorrichtung nach einem der vorstehenden Ansprüche, bei der das Guanidin ausgewählt wird aus einer Klasse, die aus einem Monoguanidin der Gruppe:

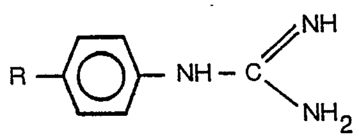

besteht, worin R ausgewählt wird aus einer Klasse, die besteht aus:

(a) einer Kette von Kohlenstoffatomen ohne andere Elemente;
(b) einer Kohlenstoffkette mit wenigstens einem weiteren Element;
(c) einer aromatischen Gruppe ohne zusätzliche Kohlenstoffatome, Kohlenstoffketten wowie andere Elemente;
(d) einer aromatischen Gruppe mit wenigstens einem Substituenten, der aus einer Klasse ausgewählt wird, die aus Kohlenstoffatomen, Kohlenstoffketten und anderen Elementen besteht;
(e) einer zyklischen nichtaromatischen Gruppe, die keine zusätzlichen Kohlenstoffatome, Kohlenstoffketten und andere Elemente enthält;
(f) einer zyklischen nichtaromatischen Gruppe mit wenigstens einem Substituenten, der aus einer Klasse ausgewählt wird, die aus Kohlenstoffatomen, Kohlenstoffketten und anderen Elementen besteht; und
(g) eine Kombination von wenigstens zwei R aus (a), (b), (c), (d), (e) und (f); und worin

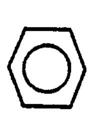

einen Benzolring darstellet;
und/oder wobei das Guanidin ausgewählt wird aus einer Klasse, die aus aromatischen Diguanidinen der Gruppe

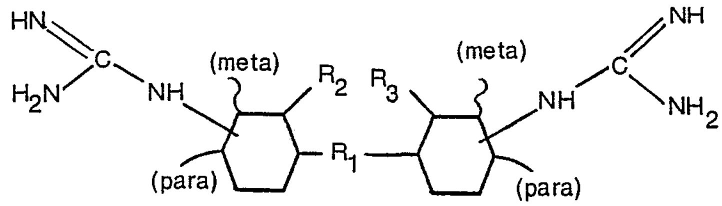

besteht, worin jede Guanidingruppe

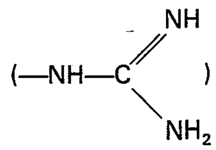

in einer Meta- oder Paraposition gegenüber von $R_1$ sich befindet, und worin $R_1$ ausgewählt wird aus einer Klasse, die besteht aus

(a) einer Kohlenwasserstoffkette, die keine Äther- oder Esterbindungen mit dem Benzolring aufweist; und
(b) einer Kohlenwasserstoffkette, die wenigstens eine Bindung aufweist, die aus einer Klasse ausgewählt wird, die aus Ätherbindungen und Esterbindungen an den Benzolring besteht; wobei

$R_2$ und $R_3$ aus einer Klasse ausgewählt werden, die besteht aus:
Wasserstoff, Chlor, Brom, Jod, einer Hydroxylgruppe und einer Alkylgruppe; und

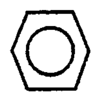

einen Benzolring darstellt; und/oder das Guanidin ein nichtaromatisches Monoguanidin der Gruppe

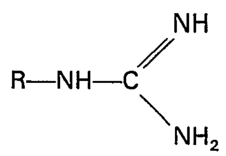

ist, worin R ausgewählt wird aus einer Klasse, die besteht aus

(a) einer Kette von Kohlenstoffatomen ohne weitere Elemente;
(b) einer Kohlenstoffkette mit wenigstens einem weiteren Element;
(c) einer zyklischen nichtaromatischen Gruppe ohne zusätzliche Kohlenstoffatome, Kohlenstoffketten und andere Elemente;
(d) einer zyklischen nichtaromatischen Gruppe mit wenigstens einem Substituenten, der aus einer Klasse ausgewählt wird, die aus Kohlenstoffatomen, Kohlenstoffketten und anderen Elementen besteht;
(e) einer Kombination von wenigstens zwei R aus (a), (b), (c) und (d); und/oder

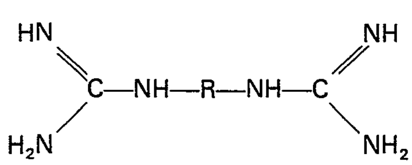

ist, worin R ausgewählt wird aus einer Klasse, die besteht aus:

(a) einer Kette von Kohlenstoffatomen ohne andere Elemente;
(b) einer Kohlenstoffkette mit wenigstens einem anderen Element;
(c) einer zylklischen nichtaromatischen Gruppe, die keine weiteren Kohlenstoffatome, Kohlenstoffketten und andere Elemente aufweist;
(d) einer zylkischen nichtaromatischen Gruppe mit wenigstens einem Substituenten, der ausgewählt wird aus einer Klasse, die aus Kohlenstoffatomen, Kohlenstoffketten und anderen Elementen besteht;

(e) einer Kombination von wenigstens zwei R aus (a), (b), (c) und (d); und/oder

das Arzneimittel ausgewählt wird aus einer Klasse, die besteht aus

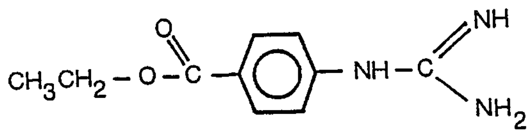

Ethyl-p-guanidinobenzoat,

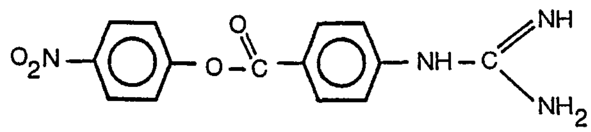

p-Nitrophenyl-p'-guanidinobenzoat (bekannt als NPGB),

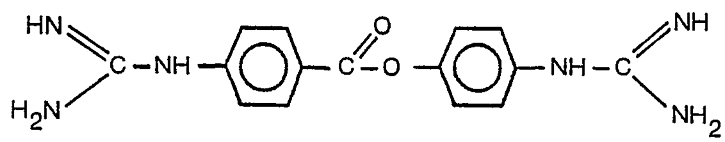

p-Guanidinophenyl-p'-guanidinobenzoat,

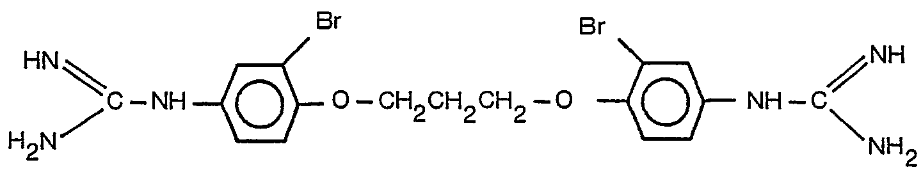

1,3-Bis (2-bromo-4-guanidinophenoxy) propan,

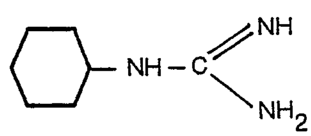

Guanidinocyclohexan, und

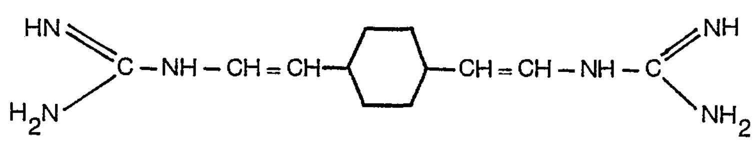

5. Eine ein Medikament aufweisende intra-uterine Vorrichtung nach einem der Ansprüche 1 bis, 4, dadurch gekennzeichnet, daß das Arzneimittel aus einer Klasse ausgewählt wird, die aus aromatischen Diaminen der Gruppe

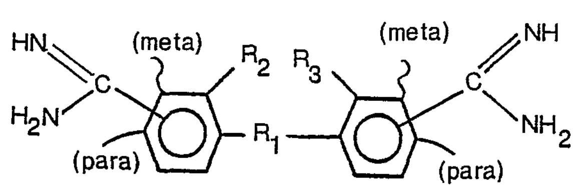

besteht, worin $R_1$ aus einer Gruppe ausgewählt wird, die aus $C_xH_y$ besteht; und $R_2$ und $R_3$ aus einer Gruppe ausgewählt werden, die aus Wasserstoff, Chlor, Brom, Jod, einer Hydroxylgruppe und einer Alkylgruppe besteht; wobei

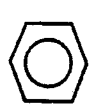

einen Benzolring darstellt.

6. Eine ein Medikament aufweisende intra-uterine Vorrichtung nach einem der Ansprüche 1 bis 4, wobei das Arzneimittel aus einer Klasse ausgewählt wird, die besteht aus 3,8-Di(*m*-amidinophenyl-diazoamino)-5-ethyl-6-phenylphenanthridinium-chlorid-Dihydrochlorid-Hydrat 8-m (m-Aminophenyl-diazoamino-3-amino-5-ethyl-6-phenylphananthridinium-chlorid,

1,4-Di (p-amidinophenoxy)-cyclohexan, und

1,4-Di (2 amidinovinyl)cyclohexan.

7. Eine ein Medikament aufweisende intra-uterine Vorrichtung nach Anspruch 6, wobei das Amidin aus einer Klasse ausgewählt wird, die aus aromatischen Monoamidinen, aromatischen Diamidinen und nichtaromatischen Diamidinen besteht.

**Revendications**

1. Dispositif intra-utérin médicamenté, caractérisé en ce que la médicament est un substance médicinale comprenant un ou plusieurs composés tels qu'une mono-amidine aromatique, une diamidine aromatique, une diamidine non aromatique, une guanidine ou un ester ou un sel correspondant, ladite substance médicinale pouvant être libérée à une vitesse de 50 à 200 $\mu$g par jour et la quantité libérée étant apte à produire un effet anti-protéolytique, anti-fibrinolytique et contraceptif.

2. Dispositif intra-utérin médicamenté suivant la revendication 1, dans lequel ladite substance médicinale comprend en outre une substance médicinale choisie dans la classe constituée par:

(a) un mélange d'une amidine et d'une guanidine;
(b) un mélange de plus d'une amidine et d'une guanidine;
(c) un mélange d'une amidine et de plus d'une guanidine;
(d) un mélange de plus d'une amidine et de plus d'une guanidine;
(e) une guanidine; et
(f) un mélange de plus d'une guanidine.

3. Dispositif intra-utérin médicamenté suivant les revendications 1 ou 2, dans lequel ladite guanidine est choisie dans la classe constituée par:

(a) des monoguanidines aromatiques;
(b) des diguanidines aromatiques;
(c) des monoguanidines non aromatiques; et
(d) des diguanidines non aromatiques.

4. Dispositif intra-utérin médicamenté suivant l'une quelconque des revendications précédentes, dans lequel ladite guanidine est choisie dans la classe comprenant une monoguanidine du groupe:

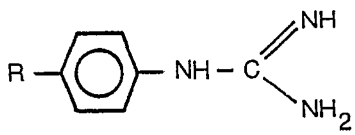

dans lequel R est choisi dans la classe comprenant:

(a) une chaîne carbonée dépourvue d'autres éléments;
(b) une chaîne carbonée comportant au moins un autre élément;
(c) un groupe aromatique dépourvu d'atomes additionnels de carbone, de chaînes carbonées et d'autres éléments;
(d) un groupe aromatique comportant au moins une addition choisie dans la classe comprenant des atomes de carbone, des chaînes carbonées et d'autres éléments;
(e) un groupe cyclique non aromatique dépourvu d'atomes additionnels de carbone, de chaînes carbonées et d'autres éléments;
(f) un groupe cyclique non aromatique comportant au moins une addition choisie dans la classe comprenant des atomes de carbone, des chaînes carbonées et d'autres éléments; et
(g) une association d'au moins deux des options (a), (b), (c), (d), (e) et (f); et

dans lequel:

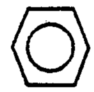

représente le noyau benzénique; et/ou ladite guanidine est choisie dans la classe comprenant des diguanidines aromatiques du groupe:

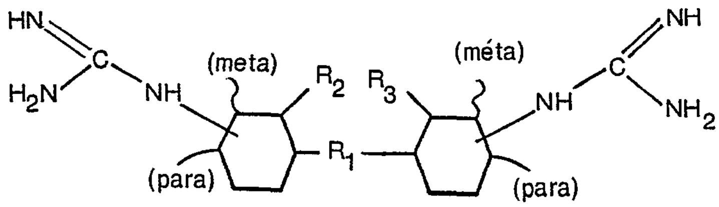

et dans lequel chaque groupe guanidino

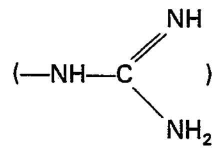

occupe l'une des positions méta ou para par rapport à $R_1$, et dans lequel:

$R_1$ est choisi dans la classe comprenant:
(a) une chaîne hydrocarbonée dépourvue de liaisons éther et ester avec le noyau benzénique; et
(b) une chaîne hydrocarbonée ayant au moins une liaison choisie dans la classe des liaisons éther et des liaisons ester avec le noyau benzénique;

$R_2$ et $R_3$ sont choisis dans la classe comprenant:
l'hydrogène, le chlore, le brome, l'iode, le groupe hydroxyle et un groupe alkyle; et

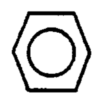

représente un noyau benzénique; et/ou ladite guanidine est une monoguanidine non aromatique du groupe:

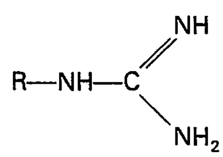

dans lequel R est choisi dans la classe comprenant:

(a) une chaîne carbonée dépourvue d'autres éléments;
(b) une chaîne carbonée présentant au moins un autre élément;
(c) un groupe cyclique non aromatique dépourvu d'atomes additionnels de carbone, de chaînes carbonées et d'autres éléments;
(d) un groupe cyclique non aromatique présentant au moins une addition choisie dans la classe comprenant des atomes de carbone, des chaînes carbonées et d'autres éléments;
(e) une association d'au moins deux des options (a), (b), (c), et (d); et/ou

ladite guanidine est une diguanidine non aromatique du groupe:

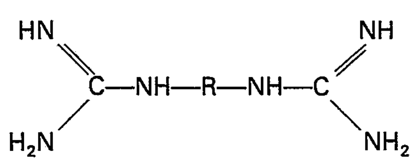

dans lequel R est choisi dans la place constituée par:

(a) une chaîne carbonée dépourvue d'autres éléments;
(b) une chaîne carbonée présentant au moins un autre élément;
(c) un groupe cyclique non aromatique dépourvu d'atomes additionnels de carbone, de chaînes carbonées et d'autres éléments;
(d) un groupe cyclique non aromatique présentant au moins une addition choisie dans la classe comprenant des atomes de carbone, des chaînes carbonées et d'autres éléments;

(e) une association d'au moins deux des options (a), (b), (c) et (d); et/ou ladite substance médicinale est choisie dans la classe comprenant:

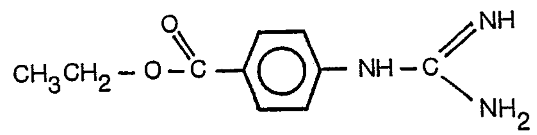

p-guanidinobenzoate d'éthyle,

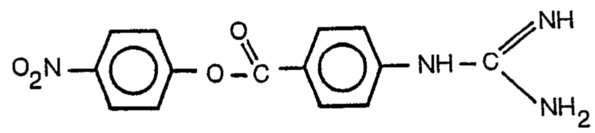

p'-guanidinobenzoate de p-nitrophényle (communément appelé NPGB),

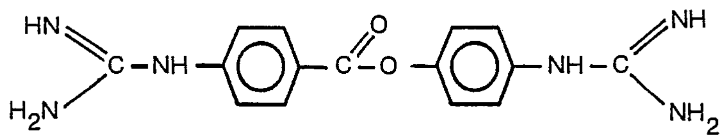

p'-guanidinobenzoate de p-guanidinophényle,

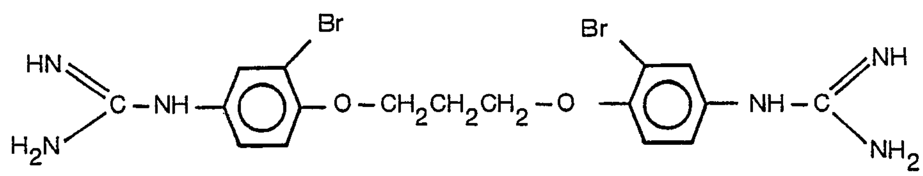

1,3-bis(2-bromo-4-guanidinophénoxy)propane,

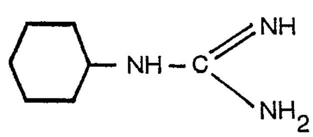

guanidinocyclohexane, et

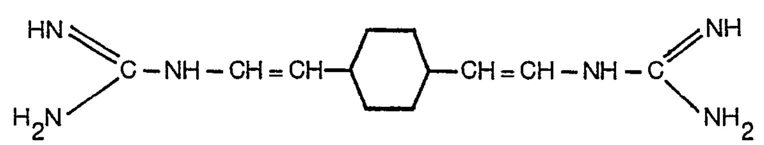

1,4-di(2-guanidinovinyl)cyclohexane.

5. Dispositif intra-utérin médicamenté suivant l'une quelconque des revendications 1 à 4, la substance médicinale étant choisie dans la classe comprenant des diamidines aromatiques du groupe

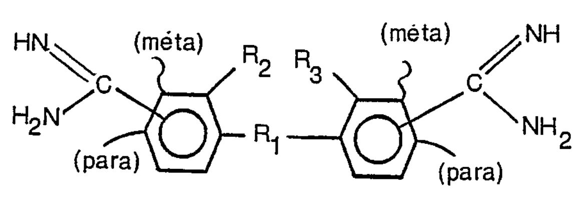

dans lequel:

$R_1$ est choisi dans le groupe comprenant $C_xH_y$; et

$R_2$ et $R_3$ sont choisis dans le groupe comprenant l'hydrogène, le chlore, le brome, l'iode, le groupe hydroxyle et des groupes alkyle; et

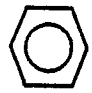

représente le noyau benzénique.

6. Dispositif intra-utérin médicamenté suivant l'une quelconque des revendications 1 à 4, la substance médicinale étant choisie dans la classe comprenant:

le dichlorhydrate de chlorure 3,8-di-(m-amidinophényldiazoamino)-5-éthyl-6-phénylphénanthridinium hydraté
le chlorure de 8-(m-amidophényldiazoamino)-3-amino-5-éthyl-6-phényl-phénanthridinium,
le 1,4-di(p-amidinophénoxy)cyclohexane, et
le 1,4-di(2-amidinovinyl)cyclohexane.

7. Dispositif intra-utérin médicamenté suivant la revendication 6, dans lequel ladite amidine est choisie dans la classe comprenant des monoamidines aromatiques, des diamidines aromatiques et des diamidines non aromatiques.